# EUROPÄISCHE PATENTANMELDUNG

(11) **EP 0 916 585 A1**
(43) Veröffentlichungstag der Anmeldung: **19.05.1999**
(21) Anmeldenummer: 98121737.5
(22) Anmeldetag: 14.11.1998
(51) Int. Cl.: B65D 25/22, B65D 25/20, A61B 19/02

(54) **Tragevorrichtung für kleinere Entsorgungsbehälter**

(30) Priorität: 18.11.1997 DE 29720424 U
(71) Anmelder: Rigling, Heinz, D-75382 Althengstett (DE)
(72) Erfinder: Rigling, Steffen, 75392 Deckenpfronn (DE)
(74) Vertreter: Steimle, Josef, Dipl.-Ing.

(57) **Zusammenfassung**

Um kleinere Entsorgungsbehälter (2) für kontaminierten Abfall, wie z. B. Kanülenboxen, deren Behälterboden eine Ausgestaltung zur lösbaren Behälterbefestigung auf einem Halter aufweist, vor allem sicherer mit nur einer Hand halten und tragen zu können, wird eine Tragevorrichtung vorgeschlagen, die wenigstens einen zumindest annähernd plattenartigen Tragekörper (1) aufweist, wenigstens mit einer zu seiner lösbaren Befestigung am Behälterboden an dessen Befestigungsausgestaltung angepaßten Ausgestaltung (5; 6, 7) und einer der Standfläche dieser Entsorgungsbehälter (2) zumindest annähernd entsprechenden Standfläche und einem vom Tragekörper (1) seitlich abstehenden Tragelement (9, 10), das für einen mobilen Einsatz der Entsorgungsbehälter (2) als ein Griff (9) und/oder als eine stationär befestigbare Halterung (10) für deren stationären Einsatz ausgestaltet ist.

## Beschreibung

Die Erfindung geht aus von kleineren, vorrangig zur mobilen Benutzung durch einzelne Personen an wechselnden Einsatzorten vorgesehenen Entsorgungsbehältern, wie sie z. B. aus dem DE-G 91 12 440.9 bekannt sind.

Diese bekannten, mit einem Deckel mit einer verschließbaren Einwurföffnung verschlossenen Entsorgungsbehälter, z. B. etwa in der Größe eines Spielzeugeimers, werden z. B. in Krankenhäusern als sogenannte Kanülenboxen von den Krankenschwestern zu den Kranken mitgenommen, um darin kontaminiertes Material, wie z. B. Kanülen von Einmalspritzen, Tupfer usw., für die kontrollierte Entsorgung sammeln zu können.

Damit diese, in bezug auf ihre Höhe schmalen und somit etwas instabilen Entsorgungsbehälter zur platzsparenden Lagerung ineinander gesteckt werden können, verjüngen sie sich konisch von oben nach unten. Dadurch haben sie eine gegenüber der Behälteröffnung kleinere Standfläche, was sich zusätzlich nachteilig auf ihre Standsicherheit auswirkt und dazu führt, daß insbesondere im annähernd gefüllten Zustand der Behälter eine Gefahr des Umkippens und ggf. Herunterfallens sowie, bei unverschlossener Einfüllöffnung, des Herausfallens von kontaminierten Material aus dem Behälter besteht.

Um dem vorzubeugen, ist es z. B. aus Krankenhäusern bekannt, diese Entsorgungsbehälter auf der Unterlage, auf der sie z. B. zum Einsatzort mitgenommen werden, das ist z. B. ein Tablett, zu befestigen. Z. B. mit Hilfe eines mittels eines Saugfußes auf dem Tablett befestigbaren Saughalters, auf dem der Behälter, z. B. durch eine entsprechende Ausgestaltung des Behälterbodens, lösbar befestigt werden kann, wie es z. B. ebenfalls aus dem DE-G 91 12 440.9 bekannt ist.

Dadurch kann jedoch die Gefahr des Umkippens, vor allem aber auch des Aus-der-Hand-Gleitens dieser Entsorgungsbehälter nicht ganz beseitigt werden. Z. B. dann nicht, wenn sie im gefüllten Zusand, in dem sie ein Gewicht von bis zu 2 kg aufweisen können, wieder von einem solchen Halter abgenommen und dabei in die Hand genommen werden müssen.

Ursache hierfür ist nicht nur die glatte Oberfläche dieser aus Kunststoff hergestellten Behälter, sondern vor allem auch die Größe ihres zudem nach oben zunehmenden Durchmessers, weshalb sie zumeist nicht einmal bis zur Hälfte ihres Umfangs mit einer Hand, zumal der einer Krankenschwester, umgriffen werden können. Bei Unachtsamkeit können sie daher aus der Hand gleiten und herunterfallen, insbesondere bei feuchten oder auch bei z. B. mit einer Schutzcreme eingecremten Händen.

Der Erfindung liegt das Problem zugrunde, die Benutzung von Entsorgungsbehältern der eingangs erläuterten Art mit einer vorrangig im Behälterboden ausgebildeten Befestigungsmöglichkeit sicherer zu machen, daß sie auch dann, wenn sie nicht zur Mitnahme auf einer mobilen Unterlage befestigt sind, zumindest auf eine Weise gehandhabt, d. h. aufgenommen, gehalten und getragen werden können, die der Gefahr des Aus-der-Hand-Gleitens bzw. des Umkippens und Herunterfallens entgegenwirkt. Dabei soll es jedoch weiterhin möglich sein, diese Behälter auf Haltevorrichtungen, wie z. B. den erwähnten Haltern, befestigen zu können.

Gelöst wird dieses Problem mit einer Tragevorrichtung mit den im Anspruch 1 wie auch im Anspruch 21 angegebenen Merkmalen. Vorteilhafte Ausgestaltungen der Erfindung sind Gegenstand der jeweiligen Unteransprüche.

Dadurch, daß die Tragevorrichtung gemäß Anspruch 1 wenigstens einen zumindest annähernd plattenartigen Tragekörper, wenigstens mit einer zu seiner lösbaren Befestigung am Boden dieser Entsorgungsbehälter an deren Befestigungs-Ausgestaltung angepaßten Ausgestaltung und einer der Standfläche dieser Entsorgungsbehälter zumindest annähernd entsprechenden Standfläche und ein vom Tragekörper seitlich abstehendes Tragelement aufweist, das für einen mobilen Einsatz der Entsorgungsbehälter als ein Griff und/oder als eine stationär befestigbare Halterung für deren stationären Einsatz ausgestaltet ist, wird erreicht, daß derartige Entsorgungsbehälter jederzeit bei Bedarf mit der erfindungsgemäßen Tragevorrichtung nachgerüstet werden können, um sie dann, wenn sie nicht auf einer ihre flexible Benutzung einschränkenden Haltevorrichtung befestigt sind, sicherer, aber auch bequemer als bisher handhaben zu können. Denn durch die erfindungsgemäß am für Befestigungszwecke ausgestalteten Boden von Entsorgungsbehältern befestigbare, für einen mobilen Behältereinsatz einen Griff aufweisende Tragevorrichtung können diese Behälter jetzt von jeder Person, unabhängig von der Größe ihrer Hände, mit nur einer Hand sicher z. B. von der Unterlage, auf der sie stehen, aufgenommen, gehalten und zum Einsatzort getragen bzw. mitgenommen werden, da funktionsgemäß nur ein Griff ein vollständiges Umschließen mit nur einer Hand ermöglicht. Es ist aber erst ein solches Umschließen mit einer Hand, das ein sicheres Greifen, Halten bzw. Tragen eines Gegenstandes ermöglicht, hier vor allem auch eines schweren, voll gefüllten Entsorgungsbehälters. Im Gegensatz zu einem von einer Hand evtl. nur bis zur Hälfte seines Umfangs umgreifbaren Behälter kann dieser mit einer um einen Griff geschlossenen Hand am sichersten gehalten werden, zumal wenn der Griff vorzugsweise handgerecht ausgestaltet und entsprechend an der Tragevorrichtung angeordnet ist.

Durch eine Tragevorrichtung mit einem als Griff ausgestalteten Tragelement kann nun ein damit vor seinem mobilen Einsatz ausgestatteter, auf einer Unterlage abstellter Entsorgungsbehälter von dieser sicher und bequem aufgenommen, gehalten und getragen und ebenso sicher auch wieder auf einer Unterlage abgestellt werden.

Durch eine solche Tragevorrichtung mit Griff können nun auch z. B. die sogenannten Kanülenboxen, wenn sie nicht mittels eines Halters auf einem Tablett befestigt sind, nicht nur ohne das zuvor erwähnte Sicherheitsrisiko, sondern auch noch viel flexibler benutzt werden. Denn ohne ein Tablett können diese Boxen mit der lösbar an ihnen befestigten Tragevorrichtung z. B. ganz spontan auf sichere und bequeme Weise mit nur einer Hand gegriffen, gehalten und z. B. bis unmittelbar ans Bett eines Kranken mitgenommen und dort z. B., wegen der im Vergleich zu einem Tablett viel wenig Platz beanspruchenden Stellfläche der Tragevorrichtung, unmittelbar auf der kleinen Fläche eines Nachttischschränkchens abgestellt werden, wodurch der Einsatz dieser Entsorgungsbehälter vereinfacht und somit erleichtert wird.

Weil die Tragevorrichtung aufgrund ihrer an die Stellfläche des von ihr zu tragenden Entsorgungsbehälters angepaßten Stellfläche dessen Standsicherheit nicht beeinträchtigt, dagegen durch eine demgegenüber schon geringfügige Vergrößerung erheblich verbessern kann und auch durch ihr Gewicht den Schwerpunkt des Behälters nach unten zu seinem Boden hin verlagern hilft, kann ein Entsorgungsbehälter mit daran befestigter Tragevorrichtung sicherer als bisher auf einer Unterlage abgestellt und befüllt und die Gefahr des Umkippens verringert werden.

Da die Tragevorrichtungen eine Ausgestaltung für eine lösbare Befestigung an entsprechend ausgebildeten Entsorgungsbehältern aufweisen, können sie auch jederzeit wieder von ihnen abgenommen werden, spätestens bevor die Behälter zur Entsorgung gegeben werden. Dadurch können diese Tragevorrichtung mehrfach verwendet werden, was den Anschaffungsaufwand einerseits und die Umweltbelastung durch ihrerseits nach Ausgebrauch zu entsorgende Tragevorrichtungen verringert.

Durch die Wahl eines geeigneten Werkstoffs, vorzugsweise Kunststoff, können nicht mehr wiederverwendbare Tragevorrichtungen sogar recycelt werden.

Das jederzeit mögliche Nachrüsten von entsprechend ausgestalteten Entsorgungsbehälter mit der neuartigen Tragevorrichtung bedeutet aber auch, daß die als ein Massenprodukt hergestellten Entsorgungsbehälter auch weiterhin ohne jegliche Veränderung, d. h. also ohne Änderung der kostspieligen Werkzeuge, produziert, aber auch, daß derartige schon produzierte, auf Lager genommene Entsorgungsbehälter mit dieser Tragevorrichtung nachgerüstet werden können.

Außerdem wird das Nachrüsten der Entsorgungsbehälter mit diesen Tragevorrichtungen dadurch erleichtert, daß letztere am Boden der Entsorgungsbehälter befestigt werden und somit die bestimmungsgemäße Behälterbenutzung zum Sammeln von kontaminierten Material zur Entsorgung, das hierzu durch eine Einfüllöffnung in den Entsorgungsbehälter eingeworfen wird, in keiner Weise beschränkt oder beeinträchtigt wird.

Dadurch, daß das für eine stationäre Benutzung von Entsorgungsbehältern an der Tragevorrichtung angeordnete Tragelement erfindungsgemäß auch als eine stationär befestigbare Halterung ausgebildet sein kann, wird erreicht, daß die Tragevorrichtung z. B. auch an einer Wand oder einer sonstigen, zum Anbringen einer solchen Halterung geeigneten Einrichtung, wie z. B. einem Regal oder Schrank, oder auch einem ggf. auch fahrbaren Gestell, für einen stationären Einsatz von Entsorgungsbehältern befestigt werden kann. Auf diese Weise kann ein auf einer derart befestigten Tragevorrichtung angeordneter Entsorgungsbehälter solange, bis er, z. B. zur Entsorgung, wieder von ihr abgenommen wird, besonders sicher ohne Gefahr des Umkippens und Herunterfallens zum Sammeln von kontaminierten Material an einem Ort eingesetzt werden. Denn durch eine solche ortsfest angebrachte Tragevorrichtung muß er zur bestimmungsgemäßen Benutzung nicht mehr in die Hand genommen werden, so daß die sonst damit verbundenen Gefahren nicht mehr auftreten können.

Ein solches stationäres Bereitstellen derartiger Entsorgungsbehälter kann z. B. im Krankenzimmer eines Betriebs oder in einer Arztpraxis sinnvoll sein, d. h. dort, wo ein solcher Entsorgungsbehälter immer am selben Platz benötigt wird. Dies gilt z. B. auch für einen Behältereinsatz in einem Notarzt-Rettungswagen, wo er ganz besonders ortsfest angebracht verfügbar sein sollte.

Trotz dieser mit Hilfe der ortfest anbringbaren Halterungs-Tragelemente von Tragevorrichtungen möglichen stationären Bereithaltung von Entsorgungsbehältern können die Behälter dennoch jederzeit, also auch vor dem Gefülltsein, von dem Tragekörper dieser Tragevorrichtungen wieder abgenommen werden. Z. B., um sie mit Hilfe einer anderen, nun mit einem Griff versehenen Tragevorrichtung sicher und bequem mobil weiter einzusetzen oder auf einer anderen ortfesten Tragevorrichtung, z. B. in einem anderen Raum, zu befestigen. D. h., die sie zuvor tragende, z. B. ein einziges Teil mit dem Tragekörper bildende Tragevorrichtung kann z. B. an der Wand befestigt bleiben.

Dabei ist es auch denkbar, daß sogar eine mit einem Griff als Tragelement ausgestattete Tragevorrichtung durch dessen entsprechende Ausgestaltung z. B. an einer Wand befestigt werden kann. Das sind im einfachsten Fall z. B. lediglich zwei in dem Griff parallel zu seiner vom Tragekörper abstehenden Richtung, z. B. zur Aufnahme von zwei Dübelschrauben vorgesehene Löcher. U. U. kann auch noch ein zwischen Wand und Griff anzuordnendes Zusatzteil für diese Verwendungsmöglichkeit vorgesehen werden, um eine plane Anlage des Griffs an der Wand zu ermöglichen.

Oder aber der Griff weist, z. B. in seinem Greifbereich, eine z. B. konisch sich von oben nach unten verjüngende Form auf, die sein Einsetzen in eine entsprechend gegenförmig ausgestaltete Wand- oder sonstige ortsfest angebrachte Halterung, z. B. in Form einer seitlich offenen kegelmantelförmigen Hülse, ermöglicht, um auf diese Weise eine Tragevorrichtung stationär einsetzen zu können.

In einer ersten, gewissermaßen eine Grundausführung der Tragevorrichtung darstellenden Lösungsvariante ist das Tragelement in seiner Ausgestaltung als Griff und/oder Wandhalterung jeweils unlösbar mit dem Tragekörper der Tragevorrichtung verbunden und bildet z. B. als ein einziges Teil mit diesem.

In einer zweiten Ausführungsvariante weisen das Tragelement und der Tragekörper eine Ausgestaltung für eine lösbare Verbindung miteinander auf. Dadurch kann die Tragevorrichtung gewissermaßen multifunktionell genutzt werden, indem der Tragekörper sozusagen wie ein Adapter benutzbar ist, an dem wechselweise entweder der Griff oder die stationär befestigbare Halterung befestigt werden kann. Das kann z. B. den Wechsel zwischen einer mobilen und stationären Einsatzweise von Entsorgungsbehältern vereinfachen und erleichtern, da hierzu nicht mehr die komplette, aus Tragekörper und Tragelement bestehende Tragevorrichtung von einem Entsorgungsbehälter entfernt werden muß, sondern der Tragekörper am Entsorgungsbehälter befestigt bleiben kann und nur noch das jeweils nicht benötigte Tragelement gegen das andere am Tragekörper ausgetauscht zu werden braucht. Das kann zusätzlich die Sicherheit der Handhabung der Entsorgungsbehältern erhöhen, da hierzu der Entsorgungsbehälter nicht unbedingt in der Hand gehalten werden muß. Stattdessen kann er ggf. mit dem daran befestigten, z. B. mit einem Griff verbundenen Tragekörper z. B. auf einer Unterlage stehen bleiben, wenn der Griff von ihm abgenommen werden soll. Oder es kann das z. B. als Wandhalterung ausgebildete Tragelement an der Wand befestigt bleiben, wenn es für eine mobile Benutzung des auf dem Tragekörper befestigten Entsorgungsbehälters durch ein Griff-Tragelement ersetzt werden soll. Hierzu müßte dann nur der Entsorgungsbehälter mit dem an ihm befestigten Tragekörper von der Wandhalterung gelöst werden, um anschließend den Griff am Tragekörper befestigen zu können.

Eine bevorzugte Ausführungsform hat die erfindungsgemäße Tragevorrichtung im Hinblick auf eine Benutzung mit kleineren Entsorgungsbehältern mit einem kreisrunden Boden, welcher zur lösbaren Anordnung der Behälter auf einer Haltevorrichtung, z. B. einem Saughalter, eine zentrische napfartig-kreisrunde Einziehung ins Behälterinnere aufweist, an deren von der Behälterunterseite zugänglichen Wandung gewindeartig ansteigend am Umfang verteilt abstehend ausgebildeten rippenförmigen Elementen zum Verklemmen mit entsprechenden Elementen der Haltevorrichtung ausgebildet sind.

Zur Anpassung an diese Behälterausgestaltung kann die Tragevorrichtung vorteilhaft einfach eine kreisrunde Scheibe sein mit zumindest einer diesbezüglichen Ausgestaltung für eine lösbare Befestigung am Behälterboden und zumindest annähernd dem gleichen, zur Erhöhung der Behälterstandsicherheit vorzugsweise aber auch größerem Durchmesser als der Behälterboden.

Die mit der erfindungsgemäßen Tragevorrichtung genutzte Ausgestaltung des Bodens von kleineren Entsorgungsbehältern kann aber auch statt einer Einziehung im Behälterboden eine dazu gegensinnig ausgebildete Ausstülpung des Behälterbodens nach unten sein, die von der dann zur Ausbildung einer Standfläche für den Entsorgungsbehälter über den Behälterboden nach unten überstehenden Behälterwandung überdeckt ist.

Eine kreisrunde Scheibenform kann der Tragekörper auch z. B. dann aufweisen, wenn ein auf seiner Unterseite flacher Behälterboden eine z. B. quadratische Querschnittsfläche aufweist. Entspricht dann die Größe des Scheibendurchmesser der Länge der Diagonalen des Behälterbodens, so steht jeweils ein Segment des Tragekörpers über die vier Bodenkanten über, wodurch die Behälter-Standsicherheit entsprechend erhöht wird.

Um die Stabilität des vorzugsweise im wesentlichen von einer Scheibe gebildeten Tragekörpers zu verbessern, aber auch, um die Tragelemente an einer solchen Scheibe anzuordnen, kann mit Vorteil an deren Rand ein nach unten gerichteter Kragen ausgebildet sein. Für eine vorteilhafte Standflächenvergrößerung des Entsorgungsbehälters, um ihn zusätzlich gegen Umkippen zu sichern, kann dann auf einfache Weise, also ohne hierfür einen besonderen Aufwand treiben zu müssen, der Scheibenrandkragen des Tragekörpers sich konisch nach unten erweitern.

Eine solche konische Erweiterung würde bei einem Behälter mit nach unten über den Behälterboden überstehender Wandung, über die der Scheibenrandkragen des Tragekörpers im Hinblick auf die Anordnung des Tagelements am Tragekörper nach unten überstehen würde, erst ab dem unteren Wandungsrand ausgebildet sein.

Zu ihrer lösbaren Befestigung an einem eine Einziehung oder eine Ausstülpung mit z. B. Festklemm-Mitteln aufweisenden Boden eines Entsorgungsbehälters hat die Tragevorrichtung vorzugsweise auf ihrer Oberseite einen daran angepaßt rohrstutzenartig ausgebildeten zentrischen Aufsatz zum Einsetzen in die Einziehung bzw. zum Aufnehmen der Ausstülpung. An dem Aufsatz sind je nachdem entweder an seiner Außenwandung oder Innenwandung Festklemm-Mittel zum Zusammenwirken mit denjenigen am Behälterboden vorgesehenen, vorzugsweise in Form von z. B. durch eine Drehbewegung z. B. des Tragekörpers gegenüber dem Entsorgungsbehälter aneinander festklemmbaren Rippenelementen.

Zur lösbaren Verbindung von Tragelement und Tragekörper kann auf der Unterseite des im wesentlichen als eine Scheibe ausgestalteten Tragekörpers eine entsprechende Aufnahme ausgebildet sein. Diese kann die Form einer durch eine diesbezügliche Ausnehmung im Scheibenrandkragen seitlich offene Kammer mit vorzugsweise rechteckigem Querschnitt zur verdrehsicheren Tragelement-Anordnung haben, in die das entsprechend ausgebildete Ende des Tragelements von der Seite her eingesteckt wird.

Das zur lösbaren Anordnung am Tragekörper bestimmte Ende des Tragelements kann für eine ggf. größere Stabilität der Verbindung z. B. auch die Form eines Ringes haben, der von unten in eine auf der Unterseite des scheibenförmigen Tragekörpers ausgebildete Ringnut z. B. eingerastet wird. Hierfür könnte z. B. der Randkragen der Scheibe die äußere Nutwange bilden, wobei das Tragelement durch eine Ausnehmung im Scheibenrandkragen seitlich vom Tragekörper absteht, um nicht die Standfläche des Tragekörpers zu beeinträchtigen.

In weiterer Ausgestaltung der Erfindung kann auch der Tragekörper seinerseits, sowohl in der Ausführung der Tragevorrichtung nach Anspruch 2 als auch nach Anspruch 3, ebenfalls eine Ausgestaltung für eine lösbare Befestigung zumindest auf den zum Halten von Entsorgungsbehältern bestimmten und angepaßten Haltevorrichtungen aufweisen, wie z. B. den schon erwähnten Saughaltern. Dadurch kann die Tragevorrichtung noch flexibler eingesetzt werden. Z. B. in der Weise, daß für eine Befestigung eines Entsorgungsbehälters auf einem solchen Saughalter nicht etwa erst die an dem Behälter, z. B. von einer vorausgehenden mobilen Benutzung, noch an ihm befestigte, einen Griff als Tragelement aufweisende Tragevorrichtung entfernt werden müßte, sondern nur der ggf. bis auf weiteres nicht benötigte Griff, so daß der Tragekörper dieser Tragevorrichtung selbst am Entsorgungsbehälter befestigt bleiben und zusammen mit dem auf ihm befestigten Entsorgungsbehälter, z. B. auf dem besagten Saughalter, befestigt werden kann. Aus Platzgründen könnte hierfür der Griff zwar vom Tragekörper abgenommen werden, doch das könnte dazu führen, daß er dann, wenn er wieder zum Tragen des vom Saughalter abgenommenen Behälters benötigt wird, nicht mehr vorhanden bzw. auffindbar ist.

Dagegen würde für eine solche Benutzungsweise eines zuvor stationär mit einer Tragevorrichtung an z. B. einer Wand befestigten Entsorgungsbehälters das hierfür als lösbar vom Tragekörper als Wandhalterung ausgestaltete, ggf. sperrig seitlich vom Tragkörper abstehende Tragelement an der Wand befestigt bleiben können und es müßte nur der Tragekörper vom Tragelement gelöst, z. B. zusammen mit dem Entsorgungsbehälter von ihm abgezogen werden.

Durch diese weitere Ausgestaltung des Tragekörpers können also derartige Benutzungswechsel der Tragevorrichtung erleichtert, beschleunigt und vereinfacht werden, was auch helfen kann, die Akzeptanz einer solchen neuartigen Tragevorrichtung zu erleichtern.

Für diese weitere Ausgestaltung der Tragevorrichtung würde die Höhe des Scheibenrandkragens mindestens der Höhe dieser Ausgestaltung entsprechen, wobei ggf. deren unterer Rand und der des Randkragens in einer die Standfläche des Tragekörpers bildenden Ebene liegen, damit diese Standfläche vom größeren Durchmesser des Schreibenrandkragens bestimmt wird.

Es ist auch denkbar, daß kleinere Entsorgungsbehälter außen am unteren Ende ihrer Wandung Mittel für ihre Befestigung auf einer Haltevorrichtung aufweisen. In Anpassung daran wäre dann die erfindungsgemäßen Tragevorrichtung als ein z. B. ringartiger Tragekörper ausgestaltet, in den der Entsorgungsbehälter zum Tragen mit seinem unteren Ende eingesetzt werden würde, wie es in Anspruch 21 angegeben ist.

Eine lediglich beispielhafte, d. h. keinesfalls als einschränkend zu verstehende Ausführungsform der Erfindung wird nachfolgend anhand von schematischen Zeichnungen näher erläutert.

Es zeigt:
- Fig. 1: in teilweiser Seitenansicht eine erfindungsgemäße Tragevorrichtung in geringfügig verkleinertem Maßstab mit einem im Querschnitt längs der Linie A - A in Fig. 3 gezeigten Tragekörper und in teilweise geschnittener Seitenansicht ein lösbar daran angeordnetes Tragelement in der Ausgestaltung als Griff zum Tragen von Entsorgungsbehältern für deren mobilen Einsatz,
- Fig. 2: eine Seitenansicht einer erfindungsgemäßen Tragevorrichtung in geringfügig verkleinertem Maßstab mit einem Tragekörper nach Fig. 1 und einem lösbar daran angeordneten Tragelement in der Ausgestaltung als Wandhalterung zum Tragen von Entsorgungsbehältern für deren stationären Einsatz,
- Fig. 3: eine Draufsicht auf den Tragekörper nach Fig. 1 und 2,
- Fig. 4: einen Querschnitt des Tragekörpers nach Fig. 3 entlang der Linie A - A in Fig. 3,

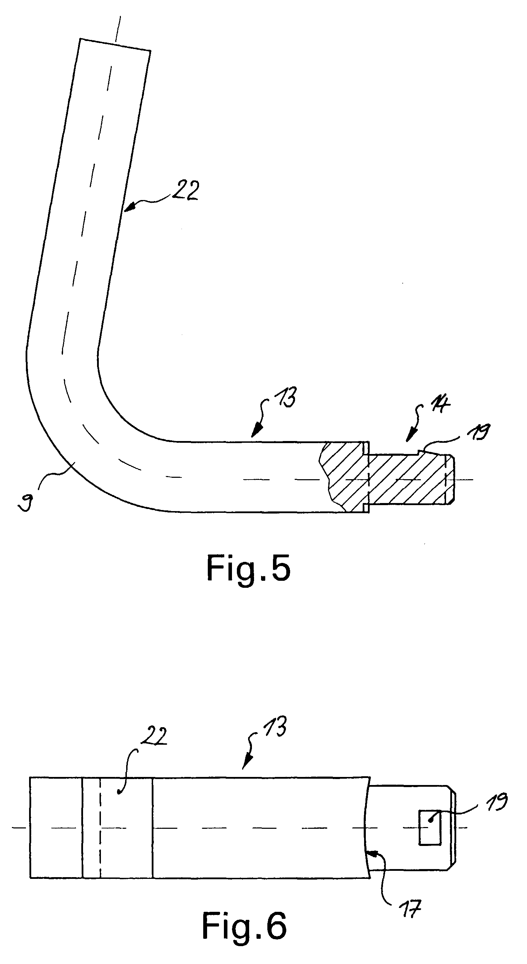

- Fig. 5: eine Seitenansicht des Griffs nach Fig. 1,
- Fig. 6: eine Draufsicht auf den Griff nach in Fig. 5,

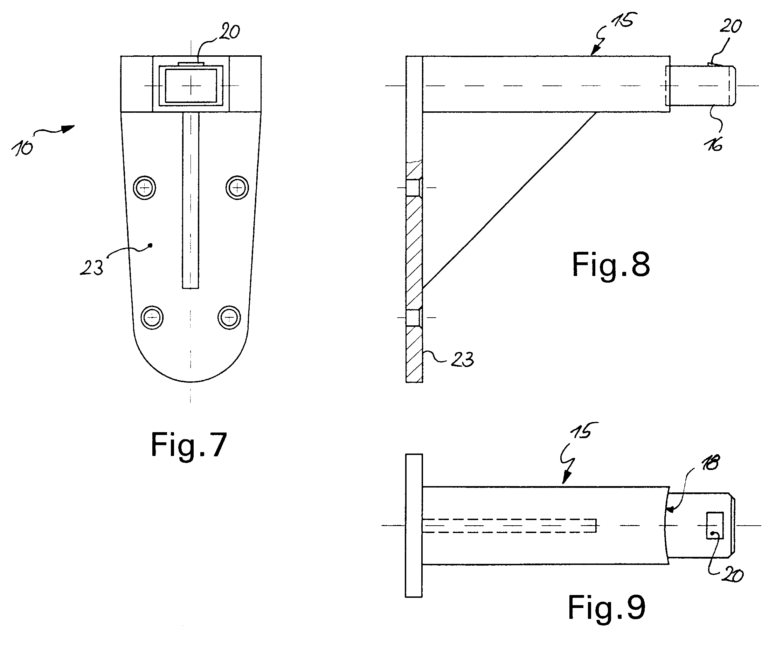

- Fig. 7: eine Vorderansicht der Wandhalterung nach Fig. 2,
- Fig. 8: eine Seitenansicht der Wandhalterung nach Fig. 2,
- Fig. 9: eine Draufsicht auf die Wandhalterung nach Fig. 2.

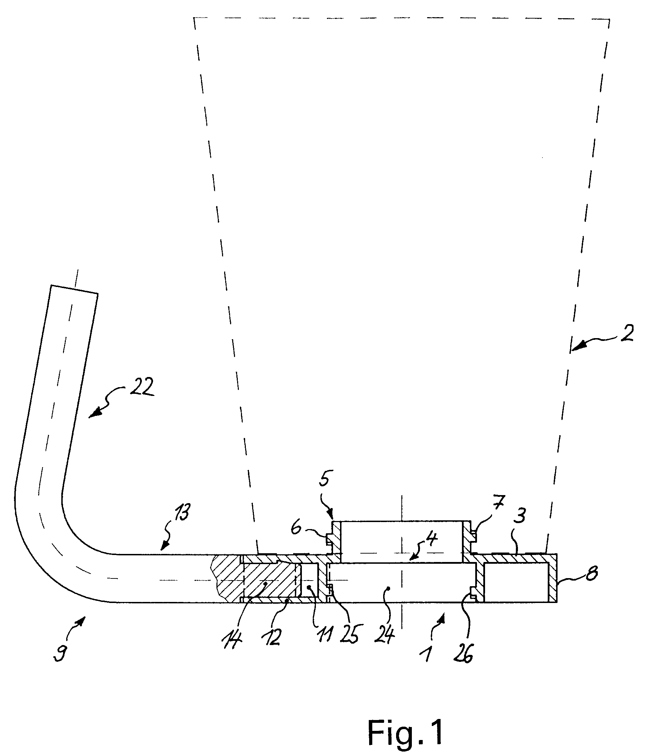

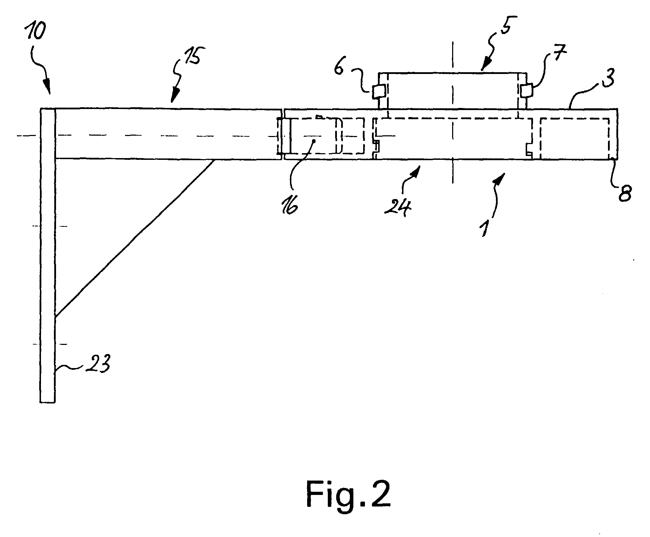

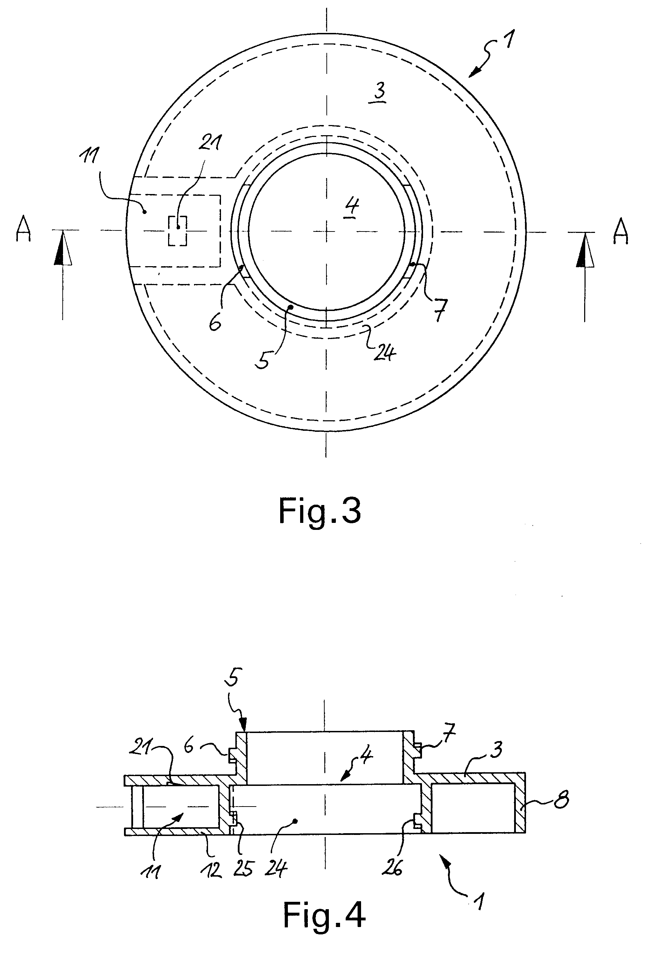

Der in Fig. 1, 2, 3 und 4 gezeigte, annähernd plattenartige Tragekörper 1 der in Fig. 1 und 2 gezeigten Tragevorrichtungen ist zur lösbaren Verbindung mit dem Boden eines in Fig. 1 gestrichelt angedeuteten Entsorgungsbehälters 2 in der ungefähren Größe eines Spielzeugeimers vorgesehen. Der kreisrund-flache Boden dieser Behälter 2 weist eine zentrische napfartige Einziehung ins Behälterinnere auf mit zwei von deren von der Bodenseite her zugänglichen Außenwandung abstehenden, einander gegenüberliegend gewindeartig ansteigend ausgebildeten Rippenelementen. Diese Ausgestaltung des Behälterbodens ist für eine lösbare Befestigung der Behälter 2 auf einer Haltevorrichtung mit einer entsprechenden gegenförmigen Ausgestaltung, z. B. in Form eines auf einer Unterlage, wie z. B. einem Tablett, befestigten Halters vorgesehen.

Gemäß der überwiegend kreisrunden Kontur der Böden von kleineren Entsorgungsbehältern 2 ist der hier gezeigte Tragekörper 1 im wesentlichen von einer Scheibe 3 mit einer zentrischen Ausnehmung 4 gebildet. Zur Vergrößerung der Standfläche des Entsorgungsbehälters, um die Gefahr des Umkippen bei abgestelltem Behälter zu verringern, ist der Durchmesser der Scheibe 3 größer als der des Bodens des Behälters 2.

Die Scheibe 3 weist auf ihrer Oberseite einen zentrisch um die Ausnehmung 4 angeordneten, rohrstutzenartigen Aufsatz 5 auf mit zum Einsetzen in die Einziehung im Boden der Entsorgungsbehälter 2 an Innendurchmesser der Einziehung angepaßten Außendurchmesser. Auf der Außenwandung des Aufsatzes 5 sind, von dieser abstehend, zwei einander gegenüberliegend angeordnete, gewindeartig ansteigend Rippenelemente 6, 7 zum Zusammenwirken mit denjenigen der Einziehung im Behälterboden ausgebildet. Das Zusammenwirken dieser beiderseitigen Rippenelemente wird z. B. durch eine Drehbewegung etwa um 90° des mit dem Aufsatz 5 in die Einziehung eingesetzten Tragekörpers 1 gegenüber dem Entsorgungsbehälter 2 herbeigeführt, wodurch sich die Rippenelemente aneinander verklemmen.

Am Rand der Scheibe 3 ist ein lediglich senkrecht nach unten von ihrer Unterseite abstehender Kragen 8 ausgebildet. Er begrenzt die Standfläche des Tragekörpers und dient gleichzeitig zur Aussteifung und Stabilisierung der Scheibe 3.

Zur lösbaren Befestigung des als Griff 9 bzw. Wandhalterung 10 ausgebildeten Tragelements des Tragekörpers 1 ist auf der Unterseite der Scheibe 3 eine kammerartige Aufnahme 11 mit rechteckigem Querschnitt mit einem einen Teil der Standfläche des Tragekörpers 1 bildenden Boden 12 ausgebildet, die durch eine entsprechende Ausnehmung im Randkragen 8 seitlich offen ist.

Fig. 1 zeigt das lösbar in die Aufnahme 11 eingesteckte, hierfür vom waagerecht verlaufenden Abschnitts 13 des Griffs 9 entsprechend abgesetzte Griff-Ende 14 und Fig. 2 das entsprechend lösbar in die Aufnahme 11 eingesteckte, vom waagerecht verlaufenden Abschnitt 15 der Wandhalterung 10 ebenfalls entsprechend abgesetzte Wandhalterungs-Ende 16, wobei beide Abschnitte 14, 16 seitlich waagerecht in radialer Richtung vom Tragekörper 1 abstehen.

Der waagerechte Abschnitt 14 des Griffs 9 hat, wie auch derjenige 16 der Wandhalterung 10, in Anpassung an den rechteckigen Querschnitt der Aufnahme 11 ebenfalls einen rechteckigen Querschnitt, wodurch er verdrehsicher in der Aufnahme 11 gehalten ist. Bei einem runden Querschnitt des Griffs müßten hierzu zusätzliche Vorkehrungen getroffen werden, z. B. in Form von in Nuten in der Wandung der Aufnahme eingreifende Rippen.

Ein in waagerechter Richtung abstehendes Tragelement erleichtert z. B. auch dessen Einstecken in und das Herausziehen aus der Aufnahme 11, z. B. in der Ausgestaltung als Griff, da hierbei der Entsorgungsbehälter mit der daran befestigten Tragevorrichtung auf der Unterlage stehen bleiben kann und nur festgehalten, nicht aber z. B. hochgehoben zu werden braucht, wodurch auch diese Maßnahme ohne die erwähnten Gefahren durchgeführt werden kann.

Durch das bündige Anschließen der in der Standflächenebene des Trageköpers 1 waagerecht verlaufenden Unterseite des ersten Abschnitts 13 des Griffs 9 an die Unterseite des Bodens 12 der Aufnahme 11 bildet er eine Verlängerung dieses Bodens 12 und somit eine seitliche Verlängerung der Standfläche des Tragekörpers 1, was sich günstig in bezug auf die Standsicherheit des auf dem Tragekörper 1 angeordneten Entsorgungsbehälters 2 auswirken kann.

Für ein bündiges Anschließen des waagerechten Abschnitts 14 des Griffs 9 bzw. desjenigen 16 der Wandhalterung 10 an die kreisrunde Kontur der Scheibe 3 weisen sie jeweils eine entsprechende Krümmung 17, 18 an den jeweiligen oberen und unteren Absatzstellen auf.

Gegen ein unkontrolliertes Lösen und Herausgleiten dieser Enden 14, 16 aus der Aufnahme 11 ist eine zweiteilige Rastsicherung vorgesehen, wovon der eine Teil als Nase 19 bzw. 20 jeweils auf der Oberseite des Griff-Endes 14 bzw. des Wandhalterungs-Endes 16 und der dazu gegenförmige andere Teil eine entsprechend keilförmige Rastnasen-Ausnehmung 21 in der Aufnahme 11 an der Unterseite der Scheibe 3 ausgebildet ist.

Wie in Fig. 1 zu sehen ist, schließt sich an den waagerecht verlaufenden Abschnitt 14 des Griffs 9 ein weiterer Abschnitt 22 an, der in einem greifgünstigen Abstand von dem auf dem Tragekörper 1 befestigten Entsorgungsbehälter 2 in einer vertikalen Ebene nach oben in Richtung zum Entsorgungsbehälter 2 hin gebogen verläuft, um so dem Griff 9 eine ergonomisch handgerechte, das Halten insbesondere eines gefüllten Entsorgungsbehälters 2 erleichternde Form zu geben.

Für ein beschwerdefreies längeres Halten des Griffs 9 kann sein zweiter Abschnitt 22 einen gerundeten Querschnitt aufweisen, ggf. mit einer an die Fingerfolge angepaßten Riffelung, um auch hier einer evtl. Gefahr des Aus-der-Hand-Gleitens dieses zweiten Griffabschnitts 22 entgegenzuwirken.

Für einen stationären Einsatz eines solchen Entsorgungsbehälters 2 kann, wie in Fig. 2 gezeigt, der Tragekörper auf das Ende 14 der z. B. an einer Wand ortsfest befestigten Wandhalterung 10 aufgeschoben werden, ohne daß hierzu der Tragekörper 1 vom Entsorgungsbehälter 2 gelöst werden müßte.

Dadurch ist jederzeit ein schneller, kein Werkzeug erfordernder Wechsel zwischer mobilem und stationären Behältereinsatz möglich, indem lediglich der Griff 9 gegen die Wandhalterung 10 oder umgekehrt getauscht wird.

Zur dauerhaften Befestigung der Wandhalterung 10 an einer Wand oder einer sonstigen dafür geeigneten Einrichtung ist der waagerechte Abschnitt der Wandhalterung 10 an einer Montageplatte 21 angeordnet.

Um auch einen auf dem Tragekörper befestigten Entsorgungsbehälter 2 jederzeit, und ohne vorher den Tragekörper 1 vom Entsorgungsbehälter 2 entfernen zu müssen, auf einer dafür bestimmten Behälter-Haltevorrichtung, z. B. einem auf z. B. einem Tablett befestigten Saughalter, befestigen zu können, ist auf der Unterseite der Scheibe 3 eine weitere, ebenfalls rohrstutzenartige, sich nach unten bis zum Rand des Scheibenrandkragens 8 erstreckende Aufnahme 24 in der gleichen Ausgestaltung wie die Einziehung des Bodens der Entsorgungsbehälter 2 vorgesehen. Sie hat folglich ebenfalls zwei gewindeartig ansteigend, einander gegenüberliegend ausgebildete, mit denjenigen der Haltevorrichtungen durch Verklemmen zusammenwirkende Rippenelemente 25, 26, wobei eine solche Aufnahme 24 zudem die Stabilität der Scheibe 3 des Tragekörpers 1 zusätzlich erhöht.

In einer gegenüber der in den Fig. 1 bis 4 gezeigten multifunktionellen oder adaptermäßigen Ausführungsform des Tragekörpers 1 können in einer einfacher ausgebildeten Ausführungsvariante der Tragevorrichtung z. B. der Griff 9 wie auch die Wandhalterung 10 jeweils unlösbar mit ihrem Tragekörper 1 verbunden, z. B. daran angeformt oder in eine aus Stabilitätsgründen der Aufnahme 11 entsprechende Verstärkung des Tragekörpers 1 z. B. auch eingeklebt sein.

Auch kann in einer einfachen Ausführungsform der Tragevorrichtung, nämlich ohne Befestigungsmöglichkeit auf einer Behälter-Haltevorrichtung, die in den Fig. 1 bis 4 gezeigte, auf der Unterseite der Scheibe 3 rohrstutzenartig ausgebildete Aufnahme 18 nicht vorgesehen sein.

## Patentansprüche

1. Tragevorrichtung für kleinere Entsorgungsbehälter, insbesondere Kanülenboxen, wobei zumindest der Boden dieser Entsorgungsbehälter eine Ausgestaltung für ihre lösbare Befestigung auf dafür vorgesehenen Haltevorrichtungen aufweist, gekennzeichnet durch wenigstens
- einen zumindest annähernd plattenartigen Tragekörper (1), wenigstens mit einer zu seiner lösbaren Befestigung am Boden dieser Entsorgungsbehälter (2) an deren Befestigungs-Ausgestaltung angepaßten Ausgestaltung (5; 6, 7) und einer der Standfläche dieser Entsorgungsbehälter (2) zumindest annähernd entsprechenden Standfläche und
- ein vom Tragekörper (1) seitlich abstehendes Tragelement (9, 10), das für einen mobilen Einsatz der Entsorgungsbehälter als ein Griff (9) und/oder als eine stationär befestigbare Halterung (10) für deren stationären Einsatz ausgestaltet ist.

2. Tragevorrichtung nach Anspruch 1, dadurch gekennzeichnet, daß das Tragelement (9, 10) unlösbar mit dem Tragekörper (1) verbunden ist.

3. Tragevorrichtung nach Anspruch 1, dadurch gekennzeichnet, daß das Tragelement (9, 10) und der Tragekörper (1) lösbar miteinander verbunden sind.

4. Tragevorrichtung nach Anspruch 2 oder 3, dadurch gekennzeichnet, daß die zum Tragen mit der Tragevorrichtung vorgesehenen Entsorgungsbehälter (2) einen Boden mit kreisrunder Kontur aufweisen und daß der Tragekörper (1) im wesentlichen von einer zumindest für die lösbare Behälterbefestigung ausgestalteten Scheibe (3) mit ebenfalls kreisrunder Kontur gebildet ist, deren Durchmesser mindestens annähernd dem Durchmesser des Bodens der Entsorgungsbehälter (2) entspricht.

5. Tragevorrichtung nach Anspruch 4, dadurch gekennzeichnet, daß die Wandung der mit der Tragevorrichtung zu tragenden Entsorgungsbehälter kragenförmig über den Behälterboden nach unten übersteht, daß zur lösbaren Behälterbefestigung auf einer Haltevorrichtung der Behälterboden eine zentrische, bis höchstens zu der vom unteren Rand der Behälterwandung aufgespannten Ebene nach unten abstehende napfartige Ausstülpung mit auf der Außenwandung gewindeartig ansteigend ausgebildeten Rippenelementen aufweist und daß der Tragekörper eine auf der Oberseite der Scheibe zentrisch angeordnete, rohrstutzenartig ausgebildete Aufnahme für die Ausstülpung des Behälterbodens aufweist mit an ihrer Innenwandung zum Zusammenwirken mit den Rippenelementen der Ausstülpung entsprechend ausgebildeten Rippenelementen.

6. Tragevorrichtung nach Anspruch 4, dadurch gekennzeichnet, daß der Boden der Entsorgungsbehälter (2) zur lösbaren Behälterbefestigung auf einer Haltevorrichtung eine zentrische napfartige Einziehung ins Behälterinnere aufweist mit auf ihrer von der Behälterunterseite zugänglichen Wandungsfläche gewindeartig ansteigend ausgebildeten Rippenelementen und daß der Tragekörper (1) einen auf der Oberseite der Scheibe (3) zentrisch angeordneten, zum Einsetzen in die Einziehung im Behälterboden angepaßten rohrstutzenartigen Aufsatz (5) aufweist mit an seiner Außenwandung zum Zusammenwirken mit den Rippenelementen der Einziehung entsprechend ausgebildeten Rippenelementen (6, 7).

7. Tragevorrichtung wenigstens nach Anspruch 6, dadurch gekennzeichnet, daß die Scheibe (3) an ihrem Rand einen nach unten gerichteten Scheibenrandkragen (8) aufweist.

8. Tragevorrichtung nach Anspruch 7, dadurch gekennzeichnet, daß sich der Scheibenrandkragen konisch nach unten erweitert.

9. Tragevorrichtung nach Anspruch 5 und 7, dadurch gekennzeichnet, daß die Höhe des Scheibenrandkragens so bemessen ist, daß er bei am Behälterboden befestigten Tragekörper über den unteren Rand der Behälterwandung übersteht.

10. Tragevorrichtung nach Anspruch 9, dadurch gekennzeichnet, daß sich der Scheibenrandkragen ab dem dem unteren Rand der Behälterwandung entsprechenden Niveau konisch nach unten erweitert.

11. Tragevorrichtung nach einem der vorhergehenden Ansprüche, dadurch gekennzeichnet, daß das Tragelement (9, 10) derart am Tragekörper (1) angeordnet ist, daß die Unterseite zumindest eines dem Tragekörper (1) benachbarten Teilabschnitts des Tragelements (9, 10) in der von der vom unteren Rand des Scheibenrandkragens (8) aufgespannten Ebene liegt.

12. Tragevorrichtung nach einem der vorhergehenden Ansprüche, dadurch gekennzeichnet, daß das Tragelement (9, 10) mit einem ersten Abschnitt (13; 15) waagerecht vom Tragekörper (1) absteht.

13. Tragevorrichtung wenigstens nach Anspruch 12, dadurch gekennzeichnet, daß das Tragelement (9) in der Ausgestaltung als Griff einen sich an den waagerecht verlaufenden Abschnitt (13) anschließenden, in einer vertikalen Ebene nach oben gerichtet verlaufenden zweiten Abschnitt (22) aufweist.

14. Tragevorrichtung nach Anspruch 13, dadurch gekennzeichnet, daß der zweite Abschnitt (22) des Griffs (9) nach einer in bezug auf den auf dem Tragekörper (1) zu befestigenden Entsorgungsbehälter (2) greifgünstigen Beabstandung nach oben zum Entsorgungsbehälter (2) hin gerichtet verläuft.

15. Tragevorrichtung nach einem der vorhergehenden Ansprüche, dadurch gekennzeichnet, daß der Griff zumindest im zweiten Abschnitt eine Ausgestaltung für eine Befestigung an einer Wand oder ortfesten Einrichtung oder an einer ortsfest angebrachten Halterung aufweist.

16. Tragevorrichtung nach einem der vorhergehenden Ansprüche, dadurch gekennzeichnet, daß bei dem Tragelement (10) in der Ausgestaltung als stationär befestigbare Halterung der waagerecht verlaufende erste Abschnitt (15) an einer Montageplatte (23) angeordnet ist.

17. Tragevorrichtung wenigstens nach einem der vorhergehenden Ansprüche, dadurch gekennzeichnet, daß zur lösbaren Anordnung eines Tragelements (9, 10) am Tragekörper (1) wenigstens eine Aufnahme (11) auf der Unterseite der Scheibe (3) für das dafür bestimmte und angepaßte Ende (14, 16) der Tragelemente (9, 10) ausgebildet ist.

18. Tragevorrichtung nach Anspruch 17, dadurch gekennzeichnet, daß die Aufnahme (11) als eine in radialer Richtung sich erstreckende, durch eine Ausnehmung im Scheibenrandkragen (8) seitlich offene Kammer ausgebildet ist.

19. Tragevorrichtung nach einem der vorhergehenden Ansprüche, dadurch gekennzeichnet, daß der Tragekörper (1) auch eine von seinen übrigen Ausgestaltungen (5, 11) unabhähgig nutzbare Ausgestaltung (24; 25, 26) für eine lösbare Befestigung auf zumindest zum Halten von Entsorgungsbehältern bestimmten und angepaßten Haltevorrichtungen aufweist.

20. Tragevorrichtung wenigstens nach Anspruch 19, dadurch gekennzeichnet, daß eine zum Halten von Entsorgungsbehältern vorgesehene Haltevorrichtung einen zylindrischen Kopf mit auf seiner Außenwandung gewindeartig ansteigend ausgebildeten Rippenelementen aufweist und daß der Tragekörper (1) eine auf der Unterseite der Scheibe (3) zentrisch angeordnete, rohrstutzenartig nach unten abstehend ausgebildete Aufnahme (24) für diesen Kopf aufweist mit an ihrer Innenwandung zum Zusammenwirken mit den Rippenelementen des Kopfes entsprechend ausgebildeten Rippenelementen (25, 26), wobei die Höhe des Scheibenrandkragens (8) wenigstens der Höhe dieser Aufnahme (24) entspricht.

21. Tragevorrichtung für kleinere Entsorgungsbehälter, insbesondere Kanülenboxen, welche zumindest Mittel für ihre lösbare Befestigung auf dafür vorgesehenen Haltevorrichtungen aufweisen, dadurch gekennzeichnet, daß derartige Befestigungsmittel wenigstens außen am unteren Ende der Behälterwandung vorgesehen sind und daß die Tragevorrichtung eine Ausgestaltung zur Aufnahme des unteren Endes des Behälters aufweist mit Befestigungsmitteln, die mit denjenigen an der Behälterwandung zusammenwirken, und mit wenigstens einem Tragelement, das für einen mobilen Einsatz der Entsorgungsbehälter als ein Griff und/oder als eine stationär befestigbare Halterung für deren stationären Einsatz ausgestaltet ist.

22. Tragevorrichtung nach Anspruch 21, dadurch gekennzeichnet, daß der Tragekörper auch eine Ausgestaltung zur lösbaren Anordnung an oder auf zumindest zum Halten von kleineren Entsorgungsbehältern bestimmten und angepaßten Haltevorrichtungen aufweist.
